# EUROPEAN PATENT SPECIFICATION

(11) **EP 3 392 341 B1**
(45) Date of publication and mention of the grant of the patent: **10.03.2021**
(21) Application number: 17382211.5
(22) Date of filing: 20.04.2017
(51) Int. Cl.: C12P 7/64, C12P 7/44, C12P 7/40

(54) **PROCESS FOR SHORTENING THE HYDROCARBON CHAIN OF A CARBOXYLIC ACID BY A PEROXYGENASE**
VERFAHREN ZUR VERKÜRZUNG DER KOHLENWASSERSTOFFKETTE EINER CARBONSÄURE DURCH EINE PEROXYGENASE
PROCÉDÉ POUR RACCOURCIR LA CHAÎNE HYDROCARBURE D'UN ACIDE CARBOXYLIQUE PAR UNE PEROXYGÉNASE

(43) Date of publication of application: 24.10.2018
(73) Proprietor: Consejo Superior de Investigaciones Científicas (CSIC), 41013 Sevilla (ES)
(72) Inventor: GUTIÉRREZ SUÁREZ, Ana, 41012 SEVILLA (ES); OLMEDO MENA-BERNAL, Andrés, 41012 SEVILLA (ES); DEL RÍO ANDRADE, José Carlos, 41012 SEVILLA (ES); MARTÍNEZ FERRER, Ángel Tomás, 28040 MADRID (ES)
(74) Representative: Pons

(56) References cited:
- WO-A1-2016/207373
- JANSEN G A ET AL: "Alpha-Oxidation", BIOCHIMICA ET BIOPHYSICA ACTA. MOLECULAR CELL RESEARCH, ELSEVIER SCIENCE PUBLISHERS, AMSTERDAM, NL, vol. 1763, no. 12, 1 December 2006 (2006-12-01), pages 1403-1412, XP027896775, ISSN: 0167-4889 [retrieved on 2006-12-01]
- VEERLE FOULON ET AL: "Breakdown of 2-Hydroxylated Straight Chain Fatty Acids via Peroxisomal 2-Hydroxyphytanoyl-CoA Lyase : A REVISED PATHWAY FOR THE [alpha]-OXIDATION OF STRAIGHT CHAIN FATTY ACIDS", JOURNAL OF BIOLOGICAL CHEMISTRY, vol. 280, no. 11, 18 March 2005 (2005-03-18), pages 9802-9812, XP055415742, ISSN: 0021-9258, DOI: 10.1074/jbc.M413362200
- HAMBERG M ET AL: "alpha-oxidation of fatty acids in higher plants. Identification of a pathogen-inducible oxygenase (piox) as an alpha-dioxygenase and biosynthesis of 2-hydroperoxylinolenic acid.", THE JOURNAL OF BIOLOGICAL CHEMISTRY, vol. 274, no. 35, 27 August 1999 (1999-08-27), pages 24503-24513, XP055415804, ISSN: 0021-9258
- ANDRÉS OLMEDO ET AL: "From Alkanes to Carboxylic Acids: Terminal Oxygenation by a Fungal Peroxygenase", ANGEWANDTE CHEMIE INTERNATIONAL EDITION, vol. 55, no. 40, 30 August 2016 (2016-08-30), pages 12248-12251, XP055415744, ISSN: 1433-7851, DOI: 10.1002/anie.201605430
- WANG YONGHUA ET AL: "Peroxygenases en route to becoming dream catalysts. What are the opportunities and challenges?", CURRENT OPINION IN CHEMICAL BIOLOGY, vol. 37, 16 December 2016 (2016-12-16), pages 1-9, XP029988258, ISSN: 1367-5931, DOI: 10.1016/J.CBPA.2016.10.007

### SEQUENCE LISTING

<110> Consejo Superior de Investigaciones Cientítifcas (CSIC)
<120> PROCESS FOR SHORTENING THE HYDROCARBON CHAIN OF A CARBOXYLIC ACID BY A PEROXYGENASE
<130> EP1641.1298
<160> 1
<170> PatentIn version 3.5
<210> 1
   <211> 236
   <212> PRT
   <213> Marasmius rotula
<400> 1
   Ser Ala His Pro Trp Lys Ala Pro Gly Pro Asn Asp Ser Arg Gly Pro
   1               5                   10                  15
   Cys Pro Gly Leu Asn Thr Leu Ala Asn His Gly Phe Leu Pro Arg Asn
               20                  25                  30
   Gly Arg Asn Ile Ser Val Pro Met Ile Val Lys Ala Gly Phe Glu Gly
           35                  40                  45
   Tyr Asn Val Gln Ser Asp Ile Leu Ile Leu Ala Gly Lys Ile Gly Met
       50                  55                  60
   Leu Thr Ser Arg Glu Ala Asp Thr Ile Ser Leu Glu Asp Leu Lys Leu
   65                  70                  75                  80
   His Gly Thr Ile Glu His Asp Ala Ser Leu Ser Arg Glu Asp Val Ala
                   85                  90                  95
   Ile Gly Asp Asn Leu His Phe Asn Glu Ala Ile Phe Thr Thr Leu Ala
               100                 105                 110
   Asn Ser Asn Pro Gly Ala Asp Val Tyr Asn Ile Ser Ser Ala Ala Gln
           115                 120                 125
   Val Gln His Asp Arg Leu Ala Asp Ser Leu Ala Arg Asn Pro Asn Val
       130                 135                 140
   Thr Asn Thr Asp Leu Thr Ala Thr Ile Arg Ser Ser Glu Ser Ala Phe
   145                 150                 155                 160
   Phe Leu Thr Val Met Ser Ala Gly Asp Pro Leu Arg Gly Glu Ala Pro
                   165                 170                 175
   Lys Lys Phe Val Asn Val Phe Phe Arg Glu Glu Arg Met Pro Ile Lys
               180                 185                 190
   Glu Gly Trp Lys Arg Ser Thr Thr Pro Ile Thr Ile Pro Leu Leu Gly
           195                 200                 205
   Pro Ile Ile Glu Arg Ile Thr Glu Leu Ser Asp Trp Lys Pro Thr Gly
       210                 215                 220
   Asp Asn Cys Gly Ala Ile Val Leu Ser Pro Glu Leu
   225                 230                 235

## Description

The invention relates to an enzymatic process for shortening the chain of a carboxylic acid with a hydrocarbon chain of at least three carbon atoms. More particularly, the invention relates to a process for one-carbon shortening the chain catalysed by a fungal peroxygenase.

### BACKGROUND ART

The use of enzymes for organic syntheses, from fine chemicals and pharmaceuticals to medium-scale and even bulk chemicals, has become an attractive alternative to chemical synthesis. Replacing organic/metal catalysts by biocatalysts has several advantages, the most important being better regio- and stereoselectivity, fewer side products, and lower environmental impact. Enzymes that catalyze the transfer of an oxygen atom from peroxide to substrates are known as peroxygenases. This subclass (EC.1.11.2) was approved in 2011 and at present comprises four members, among which the unspecific peroxygenase (UPO, EC 1.11.2.1) is the most prominent because of its versatility for oxygen transfer reactions (M. Hofrichter et al. Adv. Exp. Med. Biol., 2015, 851, 341-368) that makes these enzymes highly attractive as industrial biocatalysts.

The first UPO was discovered in the wood-dwelling basidiomycete *Agrocybe aegerita.* This peroxygenase was found to catalyze reactions formerly assigned only to P450 monooxygenases (P450s). However, unlike P450s that are intracellular enzymes whose activation often requires a flavin-containing auxiliary enzyme or protein domain and a source of reducing power, the *A*. *aegerita* UPO (AaeUPO) is a secreted protein, therefore far more stable, and only requires H₂O₂ for activation. The *A. aegerita* peroxygenase was initially shown to catalyze numerous oxygenation reactions on aromatic compounds (M. Hofrichter et al. Appl. Microbiol. Biotechnol., 2010, 87, 871-897) and, after that, the action of this enzyme on aliphatic compounds was demonstrated (A. Gutiérrez, et al. Arch. Biochem. Biophys., 2011, 514, 33-43;. Peter, M. et al, FEBS J., 2011, 278, 3667-3675) expanding its biotechnological interest and the name UPO was introduced. After this first peroxygenase, similar enzymes have also been found in the basidiomycetes *Coprinellus radians* and *Marasmius rotula,* and there are indications for their widespread occurrence in the fungal kingdom. Over one-hundred genes of the heme-thiolate peroxidase family, where UPOs are included, were identified in the analysis of 24 basidiomycete genomes (D. Floudas et al., Science, 2012, 336, 1715-1719). Among them, the *Coprinopsis cinerea* UPO (*Cci*UPO) has not been isolated from the fungus, but one of the UPO genes from its genome (protein #7249; http://genome.jgi.doe.gov/Copci1) was heterologously expressed by Novozymes A/S (Bagsvaerd, Denmark) as a biocatalyst candidate. The peroxygenase from *M. rotula* (MroUPO) shows several differences as regards to the other known peroxygenases, such as the inability to oxidize halides, less pronounced aromatic ring-oxygenating activity (G. Gröbe et al. AMB Express, 2011, 1, 31-42.) and the unique ability for terminal hydroxylation of n-alkanes (A. Olmedo et al. Angew. Chem. Int. Ed., 2016, 55, 12248-12251).

In relation to processes of one-carbon shortening hydrocarbon chain, it is known in plants, fungi and animals, the α-oxidation pathway leading to one-C shorter fatty acids (alternative to the general β-oxidation degradation, leading to two-C shorter acids). This α-oxidation pathway includes several steps (hydroxylation, activation, cleavage of the C1-C2 bond and aldehyde dehydrogenation) with several enzymes involved (V. Foulon et al., J. Biol. Chem., 2005, 280, 9802-9812; G. A. Jansen et al. Biochimica et Biophysica Acta (BBA) - Molecular Cell Research, 2006, 1763, 1403-1412). It is also known that P450 peroxygenases decarboxylate fatty acids to form n-1 terminal alkenes, but not chain-shortened fatty acids.

The present invention provides a reaction catalysed by a single enzyme for shortening the chain of a carboxylic acid, having a hydrocarbon chain (for example, fatty acids), leading to a shorter carboxylic acid. This is a carbon-by-carbon chain-shortening reaction that may be of interest for obtaining tailor-made fatty acids such as odd-numbered dicarboxylic or monocarboxylic acids (less abundant in nature than the even-number ones).

### SUMMARY OF THE INVENTION

The present invention discloses a novel process for the progressive one-carbon shortening of carboxylic acids having a hydrocarbon chain of at least three carbon atoms (besides the -COOH group), catalyzed by a single peroxygenase in the hemethiolate peroxidase/peroxygenase (HTP) superfamily, preferably by a fungal peroxygenase from *Marasmius rotula* (*Mro*UPO), in the presence of an oxidizing agent. The mechanism starts with an α-oxidation generating an α-hydroxy acid, which is further oxidized to a reactive α-keto intermediate whose decarboxylation yields the one-carbon shorter acid.

The first aspect of the present invention relates to a process for shortening the hydrocarbon chain of a compound of formula (I) comprising contacting the compound of formula (I) with a peroxide and an enzyme having peroxygenase activity and comprising an amino acid sequence with at least 80% identity to SEQ ID NO. 1, 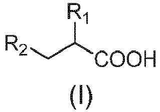 where
R₁ is selected from -H and -OH,
R₂ is selected from: optionally substituted C₁-C₁₇ alkyl, optionally substituted C₂-C₁₇ alkenyl, optionally substituted C₂-C₁₇ alkynyl, -X-COOH, 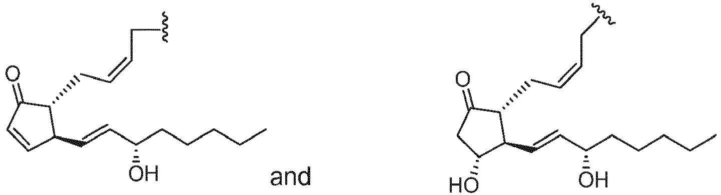
X is selected from optionally substituted C₂-C₁₆ alkyl, optionally substituted C₂-C₁₆ alkenyl, optionally substituted C₂-C₁₆ alkynyl,
wherein the peroxide is hydrogen peroxide or an organic hydroperoxide selected from the list comprising tert-butyl hydroperoxide, peracetic acid, m-chloroperoxybenzoic acid and ethyl hydroperoxide and wherein the enzymatic reaction that takes place in the above- defined process leads to at least one-carbon shorter acid, in a carbon-by-carbon shortening reaction according to the Scheme 1:

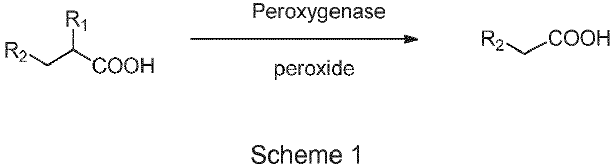

If the compound of formula (I) is a dicarboxylic acid, the main product obtained in the reaction is the one-carbon shorter dicarboxylic acid, but other secondary products can be obtained, such as the two to four-carbon shorter dicarboxylic acids, or even the α-, β- and/or γ-hydroxyderivatives of the compound of formula (I) or of the one to four-carbon shorter dicarboxylic acids.

If the compound of formula (I) is a monocarboxylic acid, the reaction occurs with lower selectivity than that with dicarboxylic acids since in addition to the chain shortening reaction, hydroxylation at the terminal (forming the dicarboxylic acids) and subterminal positions (forming the [ω-1]-keto derivatives of the monocarboxylic acids) can be produced.

Preferably, the enzyme having peroxygenase activity has an amino acid sequence having at least 90%, more preferably at least 95%, even more preferably at least 99%, sequence identity with SEQ ID NO: 1.

In the most preferably embodiment of the invention, the enzyme having peroxygenase activity is the fungal peroxygenase from *Marasmius rotula* (MroUPO) consisting of SEQ ID NO. 1 according to Protein Data Bank (PDB 5FUJ),

The SEQ ID NO. 1 is:

In a preferred embodiment, the process of the present invention is carried out in aqueous medium. Buffers can be added to the reaction medium to reach the desired pH. Preferably, the buffers are based on phosphates, such us aqueous solutions of sodium dihydrogen phosphate monohydrate. It is also possible to carry out the reaction in the desired pH without buffer with addition of acids (e.g. HCI) or bases (e.g. NaOH).

Other solvents which can be used according to the invention are protic solvents, such as methanol or ethanol, and aprotic polar solvents, such as acetone or aqueous solutions of acetone at 20%-60% (v/v), preferably at 20% acetone (v/v).

According to the invention, peroxide is hydrogen peroxide (H₂O₂) or an organic hydroperoxide selected from the list comprising *tert*-butyl hydroperoxide, peracetic acid, m-chloroperoxybenzoic acid, and ethyl hydroperoxide. More preferably, the peroxide is hydrogen peroxide.

The reaction is carried out in a range from 5°C to 50°C, preferably between 20°C and 30°C and at pH values of 3 to 10, preferably 5.5. The reaction times are in the range from 1 min to 24 h.

In a preferred embodiment of the present invention, the molar ratio between the compound of formula (I) and the enzyme having peroxygenase activity, preferably *Mro*UPO, is between 1:0.002 to 1:0.03 and/or the molar ratio between the compound of formula (I) and the peroxide is between 1:1 to 1:300.

In a preferred embodiment of the present invention, R₁ is -H.

In a preferred embodiment of the present invention, R₂ is -X-COOH, X is selected from optionally substituted C₂-C₁₆ alkyl, optionally substituted C₂-C₁₆ alkenyl, optionally substituted C₂-C₁₆ alkynyl. More preferably, R₂ is -X-COOH, where X is selected from optionally substituted C₈-C₁₀ alkyl, optionally substituted C₈-C₁₀ alkenyl, optionally substituted C₈-C₁₀ alkynyl, and even more preferable R₂ is -X-COOH where X is selected from optionally substituted C₈-C₁₀ alkyl.

In another preferred embodiment of the present invention, R₁ is -H, R₂ is -X-COOH, where X is selected from optionally substituted C₈-C₁₀ alkyl.

The dodecanedioic acid and the tetradecanedioic acid are the most preferred dicarboxylic acids of formula (I)

In another embodiment of the present invention, R₂ is selected from: optionally substituted C₁-C₁₇ alkyl, optionally substituted C₂-C₁₇ alkenyl, optionally substituted C₂-C₁₇ alkynyl. More preferably, R₂ is selected from optionally substituted C₅-C₉ alkyl, optionally substituted C₅-C₉ alkenyl, optionally substituted C₅-C₉ alkynyl and even more preferable R₂ is optionally substituted C₅-C₉ alkyl.

In another more preferred embodiment of the present invention R₁ is -H, R₂ is optionally substituted C₅-C₉ alkyl.

The decanoic acid, the α-hydroxytetradecanoic and α-hydroxydecanoic acids are preferred monocarboxylic acid of formula (I).

In another preferred embodiment of the invention, the compound of formula (I) is a prostaglandin selected from Prostaglandin E₂ and Prostaglandin A₂.

Long-chain dicarboxylic acids are versatile chemical intermediates that can be used as building blocks for polymers with new properties. This carbon-by-carbon chain-shortening reaction may be of interest for obtaining tailor-made fatty acids such as odd-numbered dicarboxylic or monocarboxylic acids (less abundant in nature than the even-number ones). The known "odd-even" effect on the aqueous solubility of dicarboxylic acids (H. Zhang et al. Ind. Eng. Chem. Res., 2013, 52, 18458-18465) could be used for product isolation, and in the synthesis of *ad-hoc* polymers. The chain-shortening decarboxylation described here is a novel reaction to be added to the repertoire of reactions that versatile fungal peroxygenases catalyze on linear and cyclic aliphatic in addition to aromatic compounds.

In the present invention, the term "C₁-C₁₇ alkyl" relates to aliphatic, linear or branched chains, more preferably linear chains, containing between 1 and 17 carbon atoms, such as for example, but not limited to, methyl, ethyl, n-propyl, i-propyl, n-butyl, tert-butyl, sec-butyl, n-pentyl, n-hexyl, n-heptyl, n-octyl, n-nonyl, n-decyl, n-undecyl, n-dodecyl, n-tridecyl, n-tetradecyl, n-pentadecyl, n-hexadecyl and n-heptadecyl. While the term "C₂-C₁₆ alkyl" relates to aliphatic, linear or branched chains, more preferably linear chains, containing between 2 and 16 carbon atoms, such as for example, but not limited to ethyl, n-propyl, i-propyl, n-butyl, tert-butyl, sec-butyl, n-pentyl, n-hexyl, n-heptyl, 1',1'-dimethylheptyl, 1,2-dimethylheptyl, 1',1'-dimethylethyl, n-octyl, n-nonyl, n-decyl, n-undecyl, n-dodecyl, n-tridecyl, n-tetradecyl, n-pentadecyl or n-hexadecyl.

The term "C₅-C₉ alkyl" relates to aliphatic, linear or branched chains, more preferably linear chains, containing between 5 and 9 carbon atoms, such as for example, but not limited to n-pentyl, n-hexyl, n-heptyl, n-octyl or n-nonyl. The term "C₈-C₁₀ alkyl" relates to aliphatic, linear or branched chains, more preferably linear chains, containing between 8 and 10 carbon atoms, such as for example, but not limited to n-octyl, n-nonyl or n-decyl.

The term "C₁-C₃ alkyl" relates to aliphatic, linear or branched chains, more preferably linear chains, containing between 1 and 3 carbon atoms such as for example, methyl, ethyl, n-propyl, i-propyl.

The term "C₂-C₁₇ alkenyl" relates, in the present invention, to linear or branched chains, more preferably linear chains, having at least one double bond and containing between 1 and 17 carbon atoms, such as for example, but not limited to vinyl, 1-propenyl, 2-propenyl, 1-butenyl, 2-butenyl, 3-butenyl, 1,3-butadienyl, 3-methyl-2-butenyl, 1-pentenyl, 1-hexenyl, 2-hexenyl, 3-hexenyl, 1-dodecenyl or similar; while the term "C₂-C₁₆ alkenyl" relates to linear or branched chains, more preferably linear chains, having at least one double bond and containing between 2 and 16 carbon atoms.

The term "C₅-C₉ alkenyl" relates to aliphatic, linear or branched chains, more preferably linear chains, having at least one double bond and containing between 5 and 9 carbon atoms, such as for example, but not limited to 1-pentenyl, 1-hexenyl, 2-hexenyl, 3-hexenyl, 1-octenyl or 1-nonenyl.

The term "C₈-C₁₀ alkenyl" relates to aliphatic, linear or branched chains, more preferably linear chains, having at least one double bond and containing between 8 and 10 carbon atoms, such as for example, but not limited to 1-octenyl, 1-nonenyl or 1-decenyl.

The term "C₂-C₁₇ alkynyl" relates, in the present invention, to linear or branched chains, more preferably linear chains, having at least one triple bond and containing between 1 and 17 carbon atoms, such as for example, but not limited to ethinyl, 1-propynyl, 2-propynyl, 2-butynyl, pentynyl, hexynyl and the like; while the term "C₂-C₁₆ alkynyl" relates to linear or branched chains, more preferably linear chains, having at least one triple bond and containing between 2 and 16 carbon atoms.

The term "C₅-C₉ alkynyl" relates to aliphatic, linear or branched chains, more preferably linear chains, having at least one triple bond and containing between 5 and 9 carbon atoms, such as for example, but not limited to 1-pentynyl, 1-hexynyl, 2-hexynyl, 3-hexynyl, 1-octynyl or 1-nonynyl.

The term "C₈-C₁₀ alkynyl" relates to aliphatic, linear or branched chains, more preferably linear chains, having at least one triple bond and containing between 8 and 10 carbon atoms, such as for example, but not limited to 1-octynyl, 1-nonynyl or 1-decynyl.

For all terms, the alkyl, alkenyl and alkynyl group may optionally be substituted by one or more substitutes such as halogen, hydroxyl, ciano, C₁-C₃ alkyl, alkoxyl, carboxyl, carbonyl, acyl, aryl, alkoxycarbonyl, amino, nitro, mercapto, alkylthio, amide, cycloalkyl, etc.

Preferably, all defined terms alkyl, alkenyl and alkynyl ("C₁-C₁₇ alkyl", "C₂-C₁₆ alkyl", "C₅-C₉ alkyl", "C₈-C₁₀ alkyl", "C₁-C₃ alkyl", "C₂-C₁₇ alkenyl", "C₂-C₁₆ alkenyl", "C₅-C₉ alkenyl", "C₈-C₁₀ alkenyl", "C₂-C₁₇ alkynyl", C₂-C₁₆ alkynyl "C₅-Cg alkynyl", "C₈-C₁₀ alkynyl") are unsubstituted.

The term "halogen" relates, in the present invention, to bromine, chlorine, iodine or fluoride.

The term "hydroxy" means the -OH group.

The term "ciano" means the -CN group.

The term "alkoxyl" is a radical of the formula -OR", wherein R" is a straight or branched alkyl group containing from 1 to 3 carbon atoms.

The term "carboxyl" is a -COOH group

The term "carbonyl" is a -CO group

The term "acyl" is a R"-CO- group, wherein R" is a straight or branched alkyl group containing from 1 to 3 carbon atoms.

The term "aryl" means an aromatic hydrocarbon group having a single ring (monocyclic) or multiple rings (bicyclic or polycyclic), which can be fused together or linked covalently.

The term "alkoxycarbonyl" is intended to indicate a radical of the formula - C(O)OR", wherein R" is a straight or branched alkyl group containing from 1 to 3 carbon atoms.

The term "amino" means the -NR'''R" group, where R'" and R" are each independently hydrogen or an straight or branched alkyl group containing from 1 to 3 carbon atoms.

The term "nitro" means the -NO₂ group.

The term "mercapto" means the -SH group.

The term "alkylthio" means the -SR" group wherein R" is a straight or branched alkyl group containing from 1 to 3 carbon atoms.

The term "amide" means the -COONH₂ group.

The term "cycloalkyl" is employed herein to represent a group of carbon-hydrogen atoms arranged in a cyclic or ring arrangement having 3 to 8 carbon atoms in the ring arrangement.

In the present invention, the term "peroxygenase activity" means an "unspecific peroxygenase" activity according to EC 1.11.2.1 , that catalyzes insertion of an oxygen atom from H₂O₂ into a variety of substrates, such as naphthalene, 4-nitrobenzodioxole; fatty acids such as tetradecanoic acid; and alkanes such as short-chain propane, hexane and cyclohexane and long-chain tetradecane. They have little or no activity toward chloride. For purposes of the present invention, peroxygenase activity is determined according to the procedure described in Gröbe, G., Ullrich, M., Pecyna, M., Kapturska, D., Friedrich, S., Hofrichter, M., Scheibner, K., 2011. High-yield production of aromatic peroxygenase by the agaric fungus Marasmius rotula . AMB Express 1, 31-42.

The term "identity" as used herein, in the context of describing two or more amino acid sequences, relates to a specified percentage of coincidences of amino acid residues at the positions from an alignment of two amino acid sequences. Sequence alignment methods for comparison are well known in the state of the art. The degree of identity can be determined using the Clustal method (Higgins, 1989, CABIOS 5: 151-153), the Wilbur-Lipman method (Wilbur y Lipman, 1983, Proceedings of the National Academy of Science USA 80: 726-730), the GAG program, including GAP (Devereux et al. 1984, Nucleic Acids Research 12: 287 Genetics Computer Group University of Wisconsin, Madison, 25 (WI)); BLAST or BLASTN, EMBOSS Needle and FASTA (Altschul et al. 1999, J. Mol. Biol. 215: 403-410). Additionally, the Smith-Waterman algorithm can also be used for the purpose of determining the degree of identity between two sequences. Preferably, the degree of identity is determined using BLAST with the default parameters. This software is publicly available at the *National Center for Biotechnology Information* (NCBI).

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

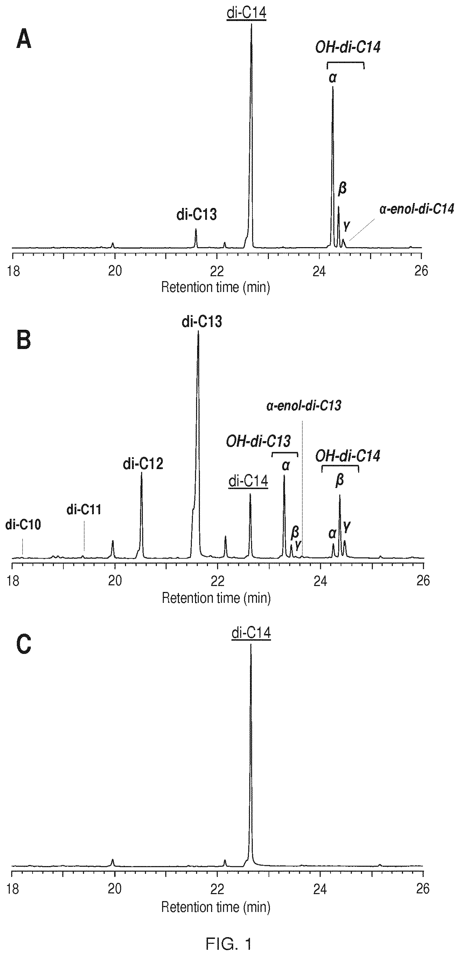

**Fig. 1****.** GC-MS analysis of reactions of tetradecanedioic acid (di-C14) with *Mro*UPO at 1 h (A), 24 h (B), and with recombinant *Coprinopsis cinerea* peroxygenase (r*Cci*UPO) at 24 h (C), showing the remaining substrate (underlined), the shortened dicarboxylic acids formed (di-C13, di-C12, di-C11 and di-C10), and the α-, β-and γ-hydroxylated derivatives (italics) of the dicarboxylic acids. Yield (%) products (A): di-C10 (<0.5), di-C11 (<0.5), di-C12 (13), di-C13 (63), OH-di-C13: α (10), β (1.4), γ (<0.5); OH-di-C14: α (2), β (7), γ (2).

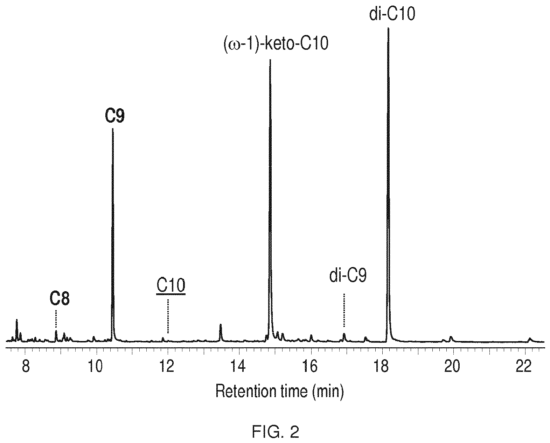

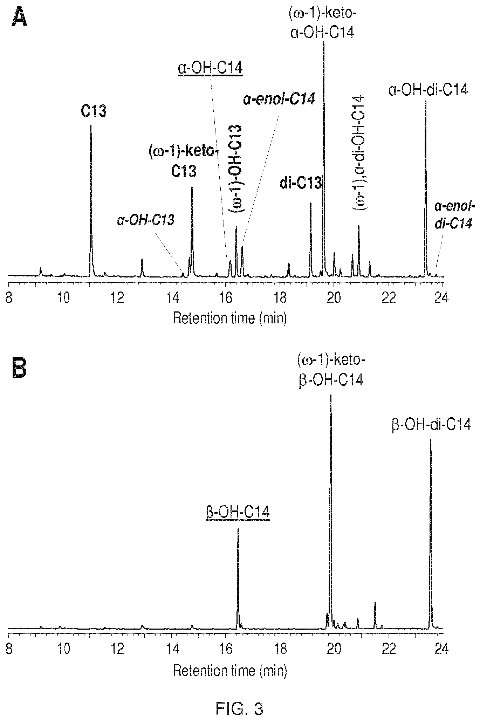

**Fig. 2**. GC-MS analysis of decanoic acid (C10) reaction with *Mro*UPO, showing the remaining substrate (underlined), the shortened monocarboxylic acids formed (C9 and C8), the α-hydroxylated form (italics) of C9, and the C10 oxygenated derivatives (keto and dicarboxylic acid). Yield (%) products: **C8** (1), **C9** (18), α-OH-C9 (2), (ω-1)-keto-C10 (34), **di-C9** (1), di-C10 (40). **Fig. 3****.** A) GC-MS analysis of *Mro*UPO reaction (2 h) with α-hydroxytetradecanoic acid, showing the remaining substrate (underlined), two α-enol forms (italics), the chain-shortened (bold) mono- (C13) and dicarboxylic (di-C13) acids and their α- and (ω-1)-oxygenated derivatives (italics), together with products from ω- and (ω-1)-oxygenation of the substrates. Yield (%) products: **C13** (18), **α-OH-C13** (0.5), **(ω-1)-keto-C13** (9), **(ω-1)-OH-C13** (5), α-enol-C14 (3), **di-C13** (7), (ω-1)-keto-α-OH-C14 (25), (ω-1),α-di-OH-C14 (5), α-OH-di-C14 (18), α-enol-di-C14 (<0.5). B) GC-MS analysis of *Mro*UPO reactions (2 h) with β-hydroxytetradecanoic.

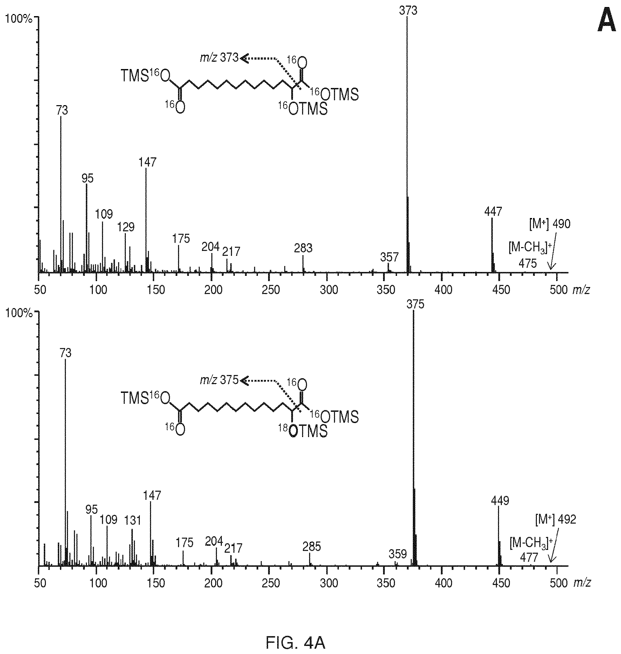

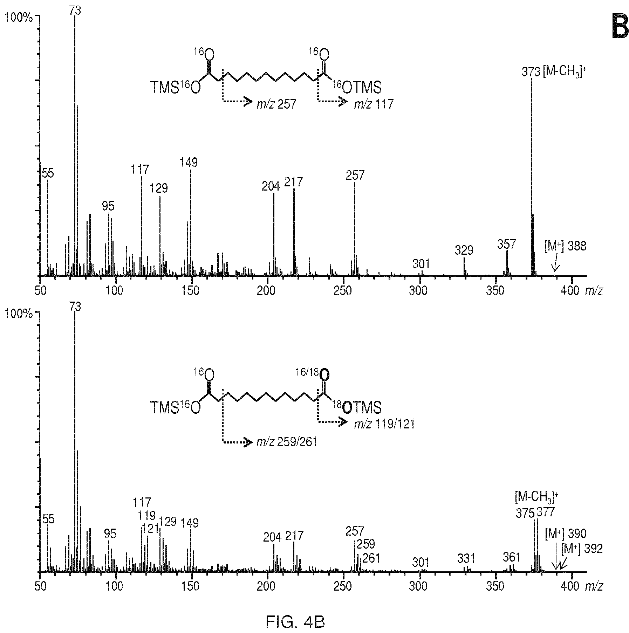

**Fig. 4** Mass spectra of α-hydroxytetradecanedioic (**A**) and tridecanedioic (**B**) acids from *Mro*UPO reactions with tetradecanedioic acid in ¹⁸O labeling experiments (bottom) and controls (top) as trimethylsilyl (TMS) derivatives, and formulae/fragmentation of the unlabeled and labeled compounds found in the H₂¹⁶O₂ (top) and H₂¹⁸O₂ (90% isotopic purity) (bottom) reactions, respectively.

### EXAMPLES

### 1. Model substrates

The reactions of the tetradecanedioic acid (dicarboxylic acid), decanoic acid (monocarboxylic acid) and α-hydroxytetradecanoic acid (a-hydroxy monocarboxylic acid), all from Sigma-Aldrich, with *Marasmius rotula* peroxygenase (*Mro*UPO) in the presence of peroxide (H₂O₂) were carry out.

To verify the assumption that chain shortening was produced via α-oxidation and not β-oxidation, the reaction of β-hydroxytetradecanoic acid (from Sigma-Aldrich) with *Mro*UPO was also studied.

To get insight into the chain shortening mechanism, ¹⁸O labeling reactions with H₂¹⁸O₂ (90% isotopic purity) were performed using tetradecanedioic acid as substrate.

### 2. Experimental details

### 2.1 Enzymes

The MroUPO enzyme is a wild-type peroxygenase isolated from cultures of *M. rotula* DSM 25031, a fungus deposited at the German Collection of Microorganisms and Cell Cultures (Braunschweig, Germany). *Mro*UPO was purified by FPLC to apparent homogeneity, confirmed by sodium dodecylsulfate-polyacrylamide gel electrophoresis under denaturing conditions, and showed a molecular mass of 32 kDa and isoelectric point of pH 5.0-5.3. The UV-visible spectrum of the enzyme showed a characteristic maximum at 418 nm (Soret band of heme-thiolate proteins), (G. Gröbe, M. Ullrich, M. Pecyna, D. Kapturska, S. Friedrich, M. Hofrichter, K. Scheibner, "High-yield production of aromatic peroxygenase by the agaric fungus Marasmius rotula" AMB Express 2011, 1, 31-42). All media and columns used for enzyme isolation were purchased from GE Healthcare Life Sciences. The *Mro*UPO enzyme consists of SEQ ID NO. 1.

The AaeUPO included in the present study for comparative purposes (*A*. *aegerita* isoform II, 46 kDa) was provided by R. Ullrich and M. Hofrichter (Technical University of Dresden, Germany) after its isolation from cultures of *A. aegerita* grown in soybean-peptone medium, and subsequent purification using a combination of Q-Sepharose and SP-Sepharose and Mono-S ion-exchange chromatographic steps (R. Ullrich, J. Nuske, K. Scheibner, J. Spantzel, M. Hofrichter, Appl. Environ. Microbiol. 2004, 70, 4575-4581).

The r*Cci*UPO, also used for comparison, was provided by Novozymes A/S (Bagsvaerd, Denmark). This recombinant enzyme corresponds to the protein model 7249 from the sequenced *C. cinerea* genome available at the JGI (http://genome.jgi.doe.gov/Copci1) expressed in *Aspergillus oryzae* (patent WO/2008/119780). The protein was purified using a combination of S-Sepharose and SP-Sepharose ion-exchange chromatography. The recombinant peroxygenase preparation is an electrophoretically homogeneous glycoprotein with a molecular mass around 44 kDa (a nonuniform glycosylation pattern was observed), a typical UV-vis spectrum with a Soret band at 418 nm, and the ability to oxygenate different aromatic compounds with a specific activity of approximately 100 U·mg⁻¹ (measured as described below).

One UPO activity unit is defined as the amount of enzyme oxidizing 1 µmol of veratryl alcohol to veratraldehyde (ε₃₁₀ 9300 M⁻¹·cm⁻¹) in 1 min at 24°C, pH 7 (the optimum for peroxygenase activity) after addition of 2.5 mM H₂O₂.

### 2.2 Enzymatic reactions

Reactions of the fatty acids (tetradecanedioic acid, decanoic acid, α-hydroxytetradecanoic acid and β-hydroxytetradecanoic acid) at 0.1 mM concentration with *Mro*UPO (0.2-3 µM) were performed in 5-mL vials containing 50 mM sodium phosphate (pH 5.5) at 30°C and 1 min-24 h reaction time, in the presence of 0.1-30 mM H₂O₂. Prior to use, the substrates were dissolved in acetone and added to the buffer to give a final acetone concentration of 20% (v/v). In control experiments, substrates were treated under the same conditions (including 2.5-15 mM H₂O₂) but without enzyme. Likewise, *Mro*UPO (0.25 µM) reactions with 0.1 mM of α-hydroxytetradecanoic acid, 20% (v/v) acetone, and 2.5 mM H₂O₂ (incubated for 2 h) were also performed. Products were recovered by liquid-liquid extraction with methyl *tert*-butyl ether and dried under N₂. *N,O-*Bis(trimethylsilyl)trifluoroacetamide (Supelco) was used to prepare trimethylsilyl (TMS) derivatives that were analyzed by GC-MS.

### 2.3 GC-MS analyses

The analyses were performed with a Shimadzu GC-MS QP2010 Ultra, using a fused-silica DB-5HT capillary column (30 m x 0.25 mm internal diameter, 0.1 µm film thickness) from J&W Scientific. The oven was heated from 50°C (1.5 min) to 90°C (2 min) at 30°C·min⁻¹, and then from 90°C to 250°C (15 min) at 8°C·min⁻¹. The injection was performed at 250°C and the transfer line was kept at 300°C. Compounds were identified by mass fragmentography, and comparing their mass spectra with those of the Wiley and NIST libraries and standards. Quantification was obtained from total-ion peak area, using response factors of the same or similar compounds. Data from replicates were averaged and, in all cases (substrate conversion and relative abundance of reaction products), the standard deviations were below 3.5% of the mean values.

### 3. Results

With respect to the reaction of the tetradecanedioic acid, under the reaction conditions used, a 93% substrate (0.1 mM) conversion by *Mro*UPO (2.3 µM) was attained after 24 h. The products include a series of chain-shortened dicarboxylic acids such as tridecanedioic (63% of products), dodecanedioic (13%), undecanedioic and decanedioic acids (the two latter in trace amounts) together with α-, β-and γ-hydroxyderivatives. (Fig. 1B). The reaction was also studied with the *C. cinerea* recombinant UPO (r*Cci*UPO) (Fig. 1C) and the well-known *A. aegerita* UPO (*Aae*UPO) (not shown), and both were unable to either catalyze the shortening of tetradecanedioic acid or even hydroxylating it.

Since both even- and odd-chain dicarboxylic acids, as well as α- and β-(and very minor amounts of γ-) hydroxyderivatives were identified with *Mro*UPO, a mechanism involving α- or γ- (for odd-chain products) or β-(for even-chain products) oxidation could be involved. At shorter (1 h) reaction time (Fig. 1A), the predominant product is α-hydroxytetradecanedioic acid, which significantly decreases after 24 h (Fig. 1B) paralleling the increase of tridecanedioic, α-hydroxytridecanedioic and dodecanedioic acids, while β-hydroxytetradecanedioic acid does not decrease. This suggests that dodecanedioic acid is formed from tridecanedioic acid *via* α-oxidation and subsequent decarboxylation, and not from tetradecanedioic acid *via* β-oxidation.

The chain shortening of fatty acids was also observed in the reaction of *Mro*UPO with the monocarboxylic fatty acid: decanoic acid, where nonanoic acid was generated (Fig. 2). However, this reaction occurred with lower selectivity than that with dicarboxylic acids since in addition to the chain shortening reaction, hydroxylation at the terminal (forming the dicarboxylic acids) and subterminal positions (forming the [ω-1]-keto derivatives of the monocarboxylic acids) was produced.

To verify the assumption that chain shortening was produced via α-oxidation and not β-oxidation, the reactions of α- and β-hydroxytetradecanoic acids with *Mro*UPO (Fig. 3) were studied. In the *Mro*UPO reaction with α-hydroxytetradecanoic acid (Fig. 3A) tridecanoic acid was one of the predominant products, while no chain shortening was produced (dodecanoic acid was not observed) from β-hydroxytetradecanoic acid (Fig. 3B), and only derivatives at the subterminal/terminal positions were identified. This demonstrates that the chain-shortening reactions, giving fatty acids with both even and odd-carbon atom numbers, happen by successive shortenings of one carbon atom via α-oxidation.

To get insight into the chain shortening mechanism, ¹⁸O labeling reactions with H₂¹⁸O₂ (90% isotopic purity) were performed using tetradecanedioic acid as substrate (Fig. 4).

The results revealed that the H₂¹⁸O₂ oxygen incorporates in tetradecanedioic acid to form α-hydroxytetradecanedioic acid, whose diagnostic fragment (*m*/*z* 373, Fig. 4A, top) appeared fully (90%) ¹⁸O-labeled (*m*/*z* 375, Fig. 4A, bottom). The ¹⁸O labeling reactions also illuminated the formation of tridecanedioic acid from tetradecanedioic acid. The incorporation of ¹⁸O was evidenced in the mass spectrum of tridecanedioic acid (Fig. 4B). In this case, the characteristic fragment at [M - CH₃]⁺ shifted from the natural abundance *m*/*z* 373 found in the unlabeled peroxide reaction (Fig. 4B, top) to *m*/*z* 377 and *m*/*z* 375 after incorporation of two or one ¹⁸O atoms, respectively (Fig. 4B, bottom). Also, the characteristic fragments at *m*/*z* 117 and *m*/*z* 257 became mono ¹⁸O-labeled (*mlz* 119 and *m*/*z* 259, respectively) and bi ¹⁸O-labeled (*mlz* 121 and *m*/*z* 261, respectively). The coexistence of mono and bi ¹⁸O-labeling of the carboxyl group suggests that a gem-diol is formed from a second α-hydroxylation of the substrate, with some hydroxyl exchange with the water (causing partial loss of ¹⁸O-labeling) before decarboxylation of its ketone form yielding the chain-shortened fatty acid, as illustrated below.

Overall, our experimental data, including ¹⁸O-labeling results, led to the peroxygenation mechanism depicted in Scheme 2. The initial product of tetradecanedioic acid (*1*) oxidation by *Mro*UPO will be the α-hydroxytetradecanedioic acid (***2***), this reaction being also reported here for the first time with a peroxygenase. Then, the product of α-hydroxytetradecanedioic acid oxidation by the peroxygenase will be a *gem-*diol (***3**, left)* from a second C_{α} hydroxylation, which will dehydrate to the ketone (**3**, *middle*) and then decarboxylate to the chain-shortened tridecanedioic acid (***4***). Direct evidence for incorporation of H₂¹⁸O₂ oxygen in the *gem*-diol/ketone formation could not be obtained since the α-ketotetradecanedioic was not detected. It is known that α-keto acids are rapidly decarboxylated by mild oxidizing reagents, such as H₂O₂ (V. Foulon, M. Sniekers, E. Huysmans, S. Asselberghs, V. Mahieu, G. P. Mannaerts, P. P. Van Veldhoven and M. Casteels, J. Biol. Chem., 2005, 280, 9802-9812; G. A. Jansen and R. J. A. Wanders, Biochimica et Biophysica Acta (BBA) - Molecular Cell Research, 2006, 1763, 1403-1412) Therefore, α-ketotetradecanedioic acid, when formed, will be decarboxylated by the H₂O₂ present in the peroxygenase reaction.

Scheme 2: Pathway for chain shortening of carboxylic acids, including dicarboxylic acids when R is a -COOH group, by *Mro*UPO, showing identified and hypothetical (normal and hydrated α-ketone) intermediates.

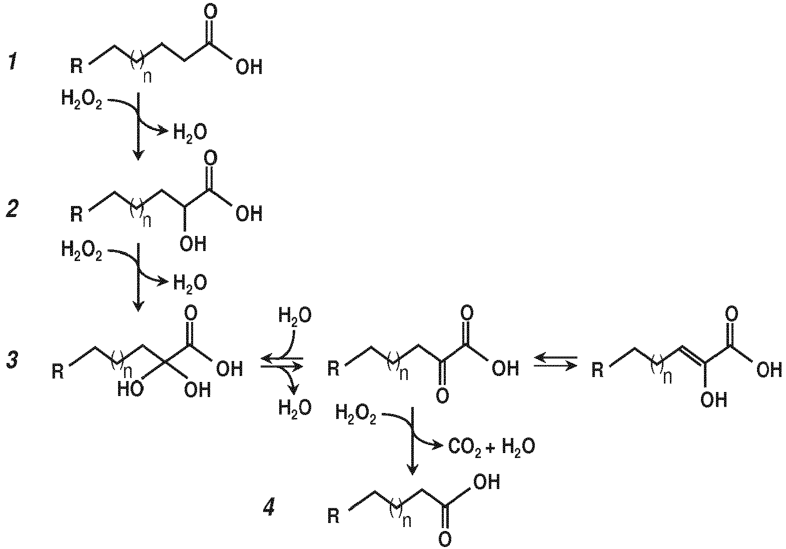

This was verified with a model compound (2-ketooctanoic acid) that, in the presence of the same H₂O₂ concentration used in the UPO reactions (2.5 mM), was decarboxylated giving heptanoic acid. Although the α-ketotetradecanedioic acid was not detected, evidence of its transient formation was obtained since its enolic form (***3***, *right*) was identified in the *Mro*UPO reactions with both 2-hydroxytetradecanoic acid (Fig. 3A) and tetradecanedioic acid (Figs. 1A-B), although in very small amounts in the latter case.

## Claims

1. A process for shortening the hydrocarbon chain of a compound of formula (I) comprising contacting the compound of formula (I) with a peroxide and an enzyme having peroxygenase activity and comprising an amino acid sequence with at least 80% identity to SEQ ID NO. 1, 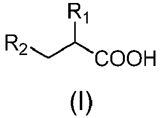 wherein
R₁ is selected from -H and -OH,
R₂ is selected from: optionally substituted C₁-C₁₇ alkyl, optionally substituted C₂-C₁₇ alkenyl, optionally substituted C₂-C₁₇ alkynyl, -X-COOH, 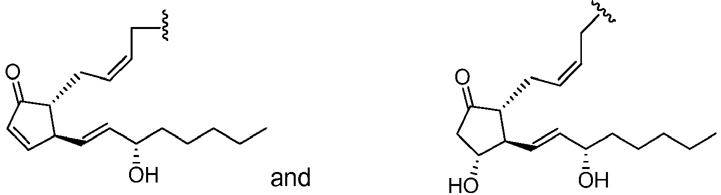
X is selected from optionally substituted C₂-C₁₆ alkyl, optionally substituted C₂-C₁₆ alkenyl, optionally substituted C₂-C₁₆ alkynyl
wherein
the peroxide is hydrogen peroxide or an organic hydroperoxide selected from the list comprising *tert-butyl* hydroperoxide, peracetic acid, *m-*chloroperoxybenzoic acid and ethyl hydroperoxide
and wherein the enzymatic reaction that takes place leads to at least one-carbon shorter acid according to the next scheme:

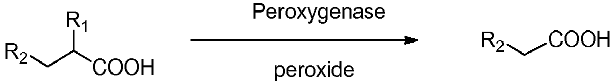

2. Process according to claim 1 wherein the enzyme having peroxygenase activity is the fungal peroxygenase from *Marasmius rotula* consisting of SEQ ID NO. 1

3. Process according to any of the preceding claims, wherein the reaction is carried out at a temperature range between 5 °C and 50 °C, preferably between 20 °C and 30 °C.

4. Process according to any of the preceding claims, wherein the pH of the reaction media is between 3 and 10, preferably the pH is 5.5.

5. Process according to any of the preceding claims, wherein the reaction is carried out in aqueous media.

6. Process according to any of the preceding claims wherein the molar ratio between the compound of formula (I) and the enzyme having peroxygenase activity is from 1:0.002 to 1:0.03 and/or the molar ratio between the compound of formula (I) and the peroxide is from 1:1 to 1:300.

7. Process according to any of the preceding claims, wherein the reaction time is between 1 min and 24 h.

8. Process according to any of the preceding claims wherein R₂ is -X-COOH, where X is selected from optionally substituted C₈-C₁₀ alkyl, optionally substituted C₈-C₁₀ alkenyl, optionally substituted C₈-C₁₀ alkynyl.

9. Process according to claim 8 wherein R₂ is -X-COOH, and X is selected from optionally substituted C₈-C₁₀ alkyl.

10. Process according to claim 9 wherein R₁ is -H and R₂ is -X-COOH, wherein X is selected from optionally substituted C₈-C₁₀ alkyl.

11. Process according to any of claims 1 to 7 wherein R₂ is selected from optionally substituted C₅-C₉ alkyl, optionally substituted C₅-C₉ alkenyl, optionally substituted C₅-C₉ alkynyl.

12. Process according to claim 11 wherein R₂ is optionally substituted C₅-C₉ alkyl.

13. Process according to claim 12 wherein R₁ is -H and R₂ is optionally substituted C₅-C₉ alkyl.

14. Process according to any of claims 1 to 7 wherein the compound of formula (I) is selected from tetradecanedioic acid, dodecanedioic acid, decanoic acid, α-hydroxytetradecanoic acid, α-hydroxydecanoic acid, Prostaglandin E₂ and Prostaglandin A₂.

## Patentansprüche

1. Verfahren zum Verkürzen der Kohlenwasserstoffkette einer Verbindung der Formel (I), umfassend das Inkontaktbringen der Verbindung der Formel (I) mit einem Peroxid und einem Enzym mit Peroxygenaseaktivität und umfassend eine Aminosäuresequenz mit mindestens 80% Identität zu SEQ ID NO. 1, 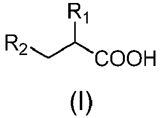 wobei
R₁ ausgewählt ist aus -H und -OH,
R₂ ausgewählt ist aus: gegebenenfalls substituiertem C₁-C₁₇-Alkyl, gegebenenfalls substituiertem C₂-C₁₇-Alkenyl, gegebenenfalls substituiertem C₂-C₁₇-Alkinyl, -X-COOH, 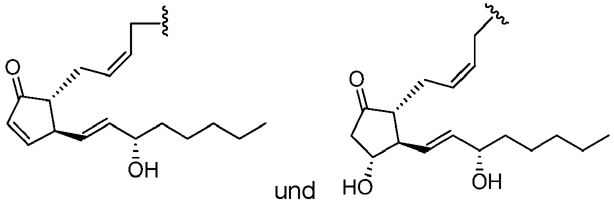
X ausgewählt ist aus gegebenenfalls substituiertem C₂-C₁₆-Alkyl, gegebenenfalls substituiertem C₂-C₁₆-Alkenyl, gegebenenfalls substituiertem C₂-C₁₆ Alkinyl, worin
das Peroxid Wasserstoffperoxid oder ein organisches Hydroperoxid ist, ausgewählt aus der Liste, umfassend *tert*-Butylhydroperoxid, Peressigsäure, m-Chlorperoxybenzoesäure und Ethylhydroperoxid
und wobei die stattfindende enzymatische Reaktion zu mindestens einer kürzeren Säure mit einem Kohlenstoff gemäß dem nächsten Schema führt:

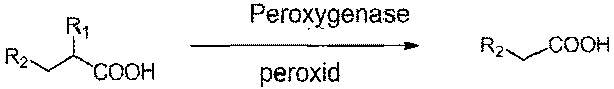

2. Verfahren nach Anspruch 1, wobei das Enzym mit Peroxygenaseaktivität die Pilzperoxygenase aus *Marasmius rotula,* bestehend aus SEQ ID NO. 1 ist.

3. Verfahren nach einem der vorhergehenden Ansprüche, wobei die Reaktion in einem Temperaturbereich zwischen 5° C und 50° C ausgeführt wird, vorzugsweise zwischen 20° C und 30° C.

4. Verfahren nach einem der vorhergehenden Ansprüche, wobei der pH-Wert des Reaktionsmediums zwischen 3 und 10 liegt, vorzugsweise der pH-Wert 5,5 ist.

5. Verfahren nach einem der vorhergehenden Ansprüche, wobei die Reaktion in wässrigen Medien ausgeführt wird.

6. Verfahren nach einem der vorhergehenden Ansprüche, wobei das Molverhältnis zwischen der Verbindung der Formel (I) und dem Enzym mit Peroxygenaseaktivität 1:0,002 bis 1:0,03 und/oder das Molverhältnis zwischen der Verbindung der Formel (I) und dem Peroxid 1:1 bis 1:300 beträgt.

7. Verfahren nach einem der vorhergehenden Ansprüche, wobei die Reaktionszeit zwischen 1 min und 24 h beträgt.

8. Verfahren nach einem der vorhergehenden Ansprüche, wobei R₂ -X-COOH ist, wobei X ausgewählt ist aus gegebenenfalls substituiertem C₈-C₁₀-Alkyl, gegebenenfalls substituiertem C₈-C₁₀-Alkenyl, gegebenenfalls substituiertem C₈-C₁₀ Alkinyl.

9. Verfahren nach Anspruch 8, wobei R₂ -X-COOH ist und X ausgewählt ist aus gegebenenfalls substituiertem C₈-C₁₀ Alkyl.

10. Verfahren nach Anspruch 9, wobei R₁ -H ist und R₂ -X-COOH ist, wobei X ausgewählt ist aus gegebenenfalls substituiertem C₈-₁₀-Alkyl.

11. Verfahren nach einem der Ansprüche 1 bis 7, wobei R₂ ausgewählt ist aus gegebenenfalls substituiertem C₅-C₉-Alkyl, gegebenenfalls substituiertem C₅-C₉-Alkenyl, gegebenenfalls substituiertem C₅-C₉ Alkinyl.

12. Verfahren nach Anspruch 11, wobei R₂ gegebenenfalls substituiertes C₅-C₉-Alkyl ist.

13. Verfahren nach Anspruch 12, wobei R₁ -H ist und R₂ gegebenenfalls substituiertes C₅-C₉-Alkyl ist.

14. Verfahren nach einem der Ansprüche 1 bis 7, wobei die Verbindung der Formel (I) ausgewählt ist aus Tetradecandisäure, Dodecandisäure, Decansäure, α-Hydroxytetradecansäure, α-Hydroxydecansäure, Prostaglandin E₂ und Prostaglandin A₂.

## Revendications

1. Procédé pour raccourcir la chaîne hydrocarbonée d'un composé de formule (I) comprenant la mise en contact du composé de formule (I) avec un peroxyde et une enzyme ayant une activité peroxygénase et comprenant une séquence d'acides aminés avec au moins 80 % d'identité avec SEQ ID NO. 1, 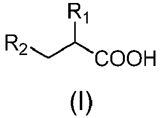 dans lequel
R₁ est choisi parmi -H et -OH,
R₂ est choisi parmi : l'alkyle en C₁-C₁₇ optionnellement substitué, l'alcényle en C₂-C₁₇ optionnellement substitué, l'alcynyle en C₂-C₁₇ optionnellement substitué, -X-COOH, 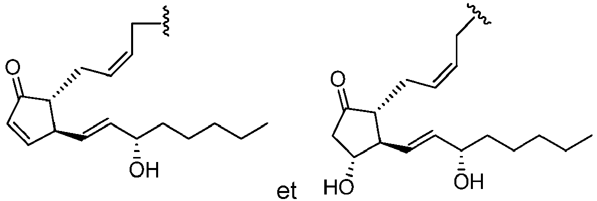
X est choisi parmi l'alkyle en C₂-C₁₆ optionnellement substitué, l'alcényle en C₂-C₁₆ optionnellement substitué, l'alcynyle en C₂-C₁₆ optionnellement substitué dans lequel
le peroxyde est le peroxyde d'hydrogène ou un hydroperoxyde organique choisi dans la liste comprenant l'hydroperoxyde de *tert*-butyle, l'acide peracétique, l'acide *m*-chloroperoxybenzoïque et l'hydroperoxyde d'éthyle
et dans lequel la réaction enzymatique qui a lieu conduit à au moins un acide monocarboné plus court selon le schéma suivant :

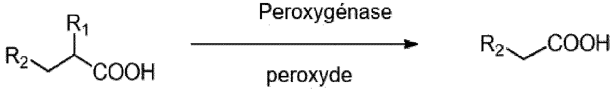

2. Procédé selon la revendication 1 dans lequel l'enzyme ayant une activité peroxygénase est la peroxygénase fongique de *Marasmius rotula* consistant en SEQ ID NO. 1.

3. Procédé selon l'une quelconque des revendications précédentes, dans lequel la réaction est effectuée à une plage de température entre 5 °C et 50 °C, de préférence entre 20 °C et 30 °C.

4. Procédé selon l'une quelconque des revendications précédentes, dans lequel le pH du milieu réactionnel est entre 3 et 10, de préférence, le pH est de 5,5.

5. Procédé selon l'une quelconque des revendications précédentes, dans lequel la réaction est effectuée dans un milieu aqueux.

6. Procédé selon l'une quelconque des revendications précédentes dans lequel le rapport molaire entre le composé de formule (I) et l'enzyme ayant une activité peroxygénase est de 1:0,002 à 1:0,03 et/ou le rapport molaire entre le composé de formule (I) et le peroxyde est de 1:1 à 1:300.

7. Procédé selon l'une quelconque des revendications précédentes, dans lequel le temps de réaction est entre 1 min et 24 h.

8. Procédé selon l'une quelconque des revendications précédentes dans lequel R₂ est -X-COOH, où X est choisi parmi l'alkyle en C₈-C₁₀ optionnellement substitué, l'alcényle en C₈-C₁₀ optionnellement substitué, l'alcynyle en C₈-C₁₀ optionnellement substitué.

9. Procédé selon la revendication 8, dans lequel R₂ est -X-COOH, et X est choisi parmi l'alkyle en C₈-C₁₀ optionnellement substitué.

10. Procédé selon la revendication 9 dans lequel R₁ est -H et R₂ est -X-COOH, dans lequel X est choisi parmi l'alkyle en C₈-₁₀ optionnellement substitué.

11. Procédé selon l'une quelconque des revendications 1 à 7 dans lequel R₂ est choisi parmi l'alkyle en C₅-C₉ optionnellement substitué, l'alcényle en C₅-C₉ optionnellement substitué, l'alcynyle en C₅-C₉ optionnellement substitué.

12. Procédé selon la revendication 11 dans lequel R₂ est l'alkyle en C₅-C₉ optionnellement substitué.

13. Procédé selon la revendication 12 dans lequel R₁ est -H et R₂ est l'alkyle en C₅-C₉ optionnellement substitué.

14. Procédé selon l'une quelconque des revendications 1 à 7 dans lequel le composé de formule (I) est choisi parmi l'acide tétradécanedioïque, l'acide dodécanedioïque, l'acide décanoïque, l'acide α-hydroxytétradécanoïque, l'acide α-hydroxydécanoïque, la prostaglandine E₂ et la prostaglandine A₂.
